(19) Europäisches Patentamt

European Patent Office

Office européen des brevets

(11) EP 0 713 715 A2

## (12) EUROPEAN PATENT APPLICATION

(43) Date of publication:
29.05.1996  Bulletin 1996/22

(51) Int. Cl.$^6$: **A61N 1/40**

(21) Application number: 95118197.3

(22) Date of filing: 19.11.1995

(84) Designated Contracting States:
DE FR GB IT

(30) Priority: 25.11.1994 IT RM940772

(71) Applicant: **Franconi, Cafiero**
I-00184 Roma (IT)

(72) Inventor: **Franconi, Cafiero**
I-00184 Roma (IT)

### (54) Shielded RF inductors for hyperthermia

(57)    The strip-inductors (5) of the disclosed hyperthermia applicator (2) are heating the tissues targets (9) with flexible heating fields generating heating cores (9') which can be optimized on the shape and size of (9). The terminals (12,13) of (5) are connected to the matching and tuning network (4) by connecting conductors (5) forming an RF tank circuit of equivalent electric length shorter than the wavelength and laying perpendicularly to the anatomic site surface (11) for limiting the effects of the electric fields of (7) and (4). The RF currents (14) flowing along (5) generate the magnetic fluxlines (15) inpinging upon (11) through air gap (8) and inducing the currents (14') within the subcutaneous muscle layer (17) and raising the therapeutic temperature of (9') without overheating the adipous layer (16). The shield (10) occludes the air gap (8) limiting the electromagnetic field leakage and improving the heating effectiveness.

FIG.1

## Description

Heating is effective in selectively killing cancerous cells in living bodies, and is a promising therapeutic modality in oncology. Heat is useful in rehabilitative treatments, in both human and veterinary clinics, concerning musculature, superficial and subcutaneous tissues and joints, and also in producing hyperhemia and the related beneficial effects such as increasing the extensibility of collagen, decreasing joint stiffness, producing pain relief, relieving muscle spasms, and assisting in the resolution of inflammatory infiltrates, edema and exudates. Further heat palliation includes the treatment of prostatic tissue, either trans-urethrally or trans-rectally with endocavitary applicators, or alse trans-abdominally with paracorporeal applicators, for relieving the simptoms of benign prostatic hyperplasia. Still further applications of heating include dispersion of adipous layers in cosmetology, and the enhancement of pharmacokinetic effects.

The invention concerns the field of hyperthermia, hereinafter referred to as HT, and is directed to the solution of problems associated with the prior art HT applicators irradiating electromagnetic energy, hereinafter referred to as EM energy for text brevity, for both paracorporeal and endocavitary tissue heating, both at microwaves, hereinafter referred to as MW, and at radiofrequency, hereinafter referred to as RF.

People expert in the art of electromagnetic HT know that an ideal paracorporeal applicator should deliver most of its energy within the first few centimetres of the muscle tissue layer, regardless of the thickness of the superficial adipous that should not be overheated for maximum comfort and safety. Moreover, it should not require any EM matching device, such as a water bolus interposed between the applicator and the body, which complicates procedures and access site monitoring of the EM energy during the treament. Also, the shape, size and conformation of the heating field should be controlled to allow personalization of the treatment plan on patients. For safety, the theoretical requirement is that the heating currents in the tissue associated to the electric field, hereinafter referred to as E-field, must substantially flow parallel to the fat-muscle interface, in order to minimize the heating of the resistive fat layer with respect to that of the conductive, and dissipative, muscle layer. In addition, the applicator should not produce unsafe levels of stray EM radiation both in the environment and inpinging perpendicularly upon the fat-muscle interface.

It is well known to people expert in electromagnetism, hereinafter referred to as experts for text brevity, that at the low RF frequencies, the heating can mainly be described in terms of RF currents rather than in terms of propagating waves. Moreover, RF applicators with a dominant inductive coupling, i.e. through the magnetic field component of the EM field, hereinafter referred to as the H-field component, represent a class of safe HT applicators, being current sources generating H-fields which are inducing high intensity beating currents in the conductive tissues such as the muscle one and weak currents in the less conductive adipous, on account that the induced currents flow substantially parallel to the fat-muscle interface. Moreover, the inductive coupling is most practical, since it occurs through an air-gap of non-critical thickness and does not require the coupling water bolus.

The prior art RF paracorporeal inductive applicators, hereinafter referred to as parallel applicators, are derived from the shortwave diathermy flat spiral placed with the loop plane of the inductive radiating structure substantially parallel to the body surface. On account of the high RF power level required and the high impedance presented by the large diameter turns, these spirals generate also substantial intra-turn E-fields impinging perpendicularly the body surface and so putting the adipous layer at risk [see: *J.Oleson, in IEEE Trans. Biomed. Engin., Vol.31, pp.91-97, 1984*]. Moreover, in the prior art diathermy systems, a tuning capacitor and the matching circuitry are positioned inside the apparatus, and are connected to the applicator inductive radiating structure with long conductors along which the intense resonance currents of the tank circuit flow, irradiating into the environment about ninety percent of the RF energy, and, therefore, heating the patient with minimal efficiency while the patients and operators are subjected to high EM risk.

Further prior art RF applicators, hereinafter referred to as perpendicular applicators, are derived from fixed geometry single-loops, in which semi-loops made up of conducting wire or sheet with the loop plane perpendicular to the body surface, terminate on a metallic backplane that has the purpose of creating a virtual image of the semi-loop carrying of the out-of-phase return current retracted at twice the distance from the body surface and minimizing the negative contribution to the heating field [*J.Bach-Andersen et al., IEEE Trans. Biomed. Eng., Vol.31, pp.21-27, 1984*]. However, this type of perpendicular applicators, and those derived from them are virtually unshielded, and are radiating a substantial part of the EM energy sideways in the environment, and therefore exhibit fixed heating field distributions of low heating effectiveness and high risk [*R.H.Johnson et al., in Proc. Hypert. Oncology 1988, T.Sugahara and M.Saito eds., pp.832-833, Taylor & Francis, 1989;C.Franconi et al., IEEE Trans. Micr. Theory Techn.,Vol. 34, pp.612-619, 1986*].

Instead, the composite heating field of the hybrid RF applicators of the prior art are accomplished by the superposition to the fixed transverse-mode EM field of a waveguide passband applicator, of the variable H-field configuration generated by a resonant inductive device integrated in the waveguide aperture and phase and amplitude controlled for positive interference as in a two-element hybrid phased array, while the tuning capacitor is positioned at the waveguide bottom [*US Patent # 5,186,181*]. At higher frequencies, prior art hybrid applicators are implemented with small rectangular cross-section waveguides on account of the smaller wavelength, and fixed field radiators extending across the whole waveguide aperture, but they appear to carry no more advantages over the prior art fixed field, aperture waveguide MW applicators generating a fixed modal field corresponding to the excited waveguide transverse mode *[ R.H. Johnson et al., Phys.Med.Biol., Vol.35, 761-779, 1990 ]*, or else over prior art linear MW dipole antennae, usually resonating at 2450 MHz, and provided of a reflector, which also are used to produce heating fields *[A.W. Guy: Biophysics of high frequency currents and EM radiation, in Therapeutic Heat and Cold, J.F.Lehmann (ed.) 1982, Williams and Wilkins, page 246]*.

Prior art endocavitary applicators are operated at MW frequencies up to 2450 MHz and are linear monopole or dipole antennae. They are narrow-band distributed-constant resonators constituted by straight conductor segments supporting longitudinal standing waves for a number of quarter wavelengths, which are inserted into the body cavity facing the target and are end-fed from the MW source through a coaxial cable segment *[US Patent 4,204,549]*. On account of the standing wave character of their resonance, their longitudinal heating pattern exhibits multiple null-points including one at the distal end of the radiator giving rise to poor tip heating *[J.W. Hand, in Interstitial Hyperthermia, L.Handl-Zeller (ed), Springer Verlag, Wien, 1992, pp. 51-75]*. Being the wavelength much shorter than the antenna-cable assembly, unwanted standing waves occur also along the cable segment and return MW currents flowing back along the cable outer shield, so that the matching and tuning device inserted at the proximal cable segment end is actually matching both cable and antenna together, with the result that there is an interdependence of the longitudinal power deposition pattern with both cable segment length and the dielectric parameters of the tissue in which is embedded, that makes the effective heating pattern length non predictable.

The same shortcomings occur with the prior art endocavitary Helix MW radiators. Helices are broadband, multi-mode MW resonators exhibiting longitudinal standing wave null-points, which depend on the helix diameter, pitch, and wire length *[K.H. Luk, et al., SAR patterns of a helical microwave intracavitary applicator, in Hyperthermic Oncology, J.Overgaard (ed), Taylor and Francis, London, 1984, pp.591-594]*. Helix radiators with circumference smaller than both axial radiator length and radiation wavelength have the dominant propagation mode (the broadside mode) in which the radiation propagates normally to the Helix axis with improved efficiency but with of a loss of penetration. *[T. Satoh et al., Int. J. Hypert., Vol.4, 497-512, 1988 ]*. Medium (5 mm) and large (20 mm) diameter Helices have been operated in the 434-2450 MHz range for HT treatments in cavities including the oesophagus *[Z.Liru et al., Int. J. Hyperth., Vol.6, 745-753, 1990, and R.Liu et al., Int.J. Hyperth., Vol.7, 577-586, 1991]*, the prostatic urethra *[M.Astrahan et al., Int.J.Hyperth.,Vol.7,pp.141-155, 1991]*, the vagina and the rectal ampoule *[D.J.Li et al., Int.J.Rad. Onc. Biol.Phys., Vol.10, 2155-2162, 1984]*, the cervix and the nasopharingeal cavity *[Q.R.Zong et al., Int. J. Hyperth.,*

*Vol.6, 997-1004, 1990, and: D.J.Li et al. , Int. J. Hyperth., Vol. 7, 693-701, 1991]*.

This invention discloses both an effective and versatile heating modality and highly safe and simple to operate applicators, to inductively deliver EM energy in the RF frequency range for either local, or loco-regional, treatments of superficial or subcutaneous targets of any sizes and shapes localized either at external anatomic sites or across the wall of body cavities, together with optimization means which also fall under the scope of the invention. The foregoing and other objects and advantages of the present invention will appear from the following description which is referred to in the accompanying drawings in which preferred embodiments of the invention are illustrated, and in which like numerals correspond to like parts. It will be apparent to any expert of EM hyperthermia that various modifications, integrations and combinations of the illustrated embodiments fall within the scope of the invention, and that the cited examples of particular embodiments are given by way of illustration and are not intended as limitations on the scope of the invention, which is susceptible of apparently widely different embodiments without departing from the scope thereof.

The preferred embodiment and best mode of the invention is schematically illustrated in

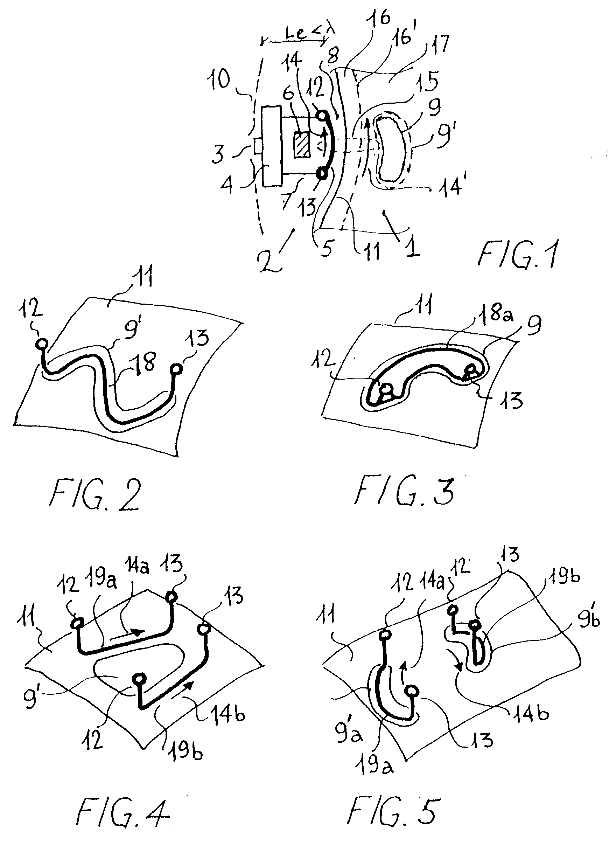

**FIG. 1** with parts cutaway.

In **FIG.s 2** and **3** some preferred strip-inductors according to the invention embodied with shaped single wire or sheet of low inductance are schematically illustrated with parts cutaway.

In **FIG.s 4** and **5** some preferred wire strip-inductor pairs according to the invention are schematically illustrated with parts cutaway embodied as two-element phased array with positive and negative interference patterns, respectively.

In **FIG. 6**, an anatomic site conformal, parallel split strip-inductor preferred embodiment according to the invention is schematically illustrated with parts cutaway.

In **FIG.s 7**, **8**, **9**, and **10** some preferred axial-inductor embodiments according to the invention are schematically illustrated with parts cutaway.

In **FIG.11**, a preferred patch-inductor embodiment according to the invention is schematically illustrated with parts cutaway.

In **FIG.12**, a preferred toroidal-inductor embodiment according to the invention is schematically illustrated with parts cutaway.

In **FIG.s 13**, **14**, **15**, preferred embodiments of the tank circuit including the matching and tuning network and single or multiple strip-inductors according to the invention for paracorporeal treatments are schematically illustrated with parts cutaway.

In **FIG.16**, a preferred embodiment of the tank circuit shaped for endocavitary heating provided of the network and of an axial strip-inductor according to the invention, is schematically illustrated with parts cutaway.

In FIG.s 17 and 18, preferred embodiments of the tank circuit for local and loco-regional paracorporeal heating according to the invention and provided of specific efficiency and regional shield embodiments according to the invention, respectively, are schematically illustrated with parts cutaway.

In FIG. 19, a preferred embodiment of the tank circuit of an endocavitary applicators according to the invention provided of a preferred EM shield embodiment according to the invention is schematically illustrated with parts cutaway.

The best modes of the invention are based on the operating principle described with the help of FIG. 1 showing a layout without details with the main components of the disclosed applicators and in which the amplitude controlled RF power source, its feeding line and the temperature monitoring unit required for HT treatments are not shown. FIG.1 shows body region (1) heated by applicator (2) fed with RF energy at the operating frequency through port (3) and matching and tuning network (4), hereinafter referred to as the network, which connects to the heating structure (5) sometimes mounted on the preferably flexible supportive platform (6). Network (4) is connected to structure (5) through conductors (7) to produce a power resonant circuit, i.e. a tank circuit, tuned at the operating frequency and matched to the impedance of the feeding line at (3). Structure (5) is an inductive device according to the invention, that is dimensioned, shaped and conformed for the optimized transfer of RF energy through air gap (8) to a core of tissue volume (9'), hereinafter referred to as the heating core or simply as core, which reaches the terapeutic temperature $T_c$. Core (9') encompasses the portion of body tissue to be exposed (9), hereinafter referred to as the target, which results heated to a temperature usually not lower than $T_c$. Shield (10) is an integral part of applicator (2), and is embodied to set a boundary for the EM field in ways functional to the applicator's clinical performance.

In order to optimize the advantages of the present invention, standing waves across the tank circuit are to be avoided. This requirement is satisfied if the wavelength is longer than the equivalent electric length $L_e$ of the tank circuit assembly, which requires operating frequencies below the MW range, i.e. in the RF range. Being $L_e$ the sum of the following equivalent electric lengths: $L_n$ of network (4), $L_c$ of the connecting means (7), and $L_s$ of strip-inductor (5), $L_e$ should be made smaller than the wavelength, a condition fulfilled by the disclosed applicators below about 450 MHz, at which frequency the wavelength is about 0.7m.

RF heating is superior to prior art MW heating in terms of longitudinal and transverse heating field pattern control, owing to the substantial independence of RF radiator shapes, sizes and conformation from the wavelength, and exhibit higher penetration potential. Structures( 5) can be implemented with highly conformable lumped or semi-lumped circuits exploiting the mature and low cost RF technology to generate flexible heating H-fields beams allowing an effective local control of the core pattern without producing significant levels of stray E-fields. Structures (5) are embodied as strips of conductors in which the inducing RF currents that they carry are substantially pure current sources producing high levels of substantially pure H-field beams, locally producing in turn core (9') heating patterns to match the size, shape and depth of targets (9) positioned in any anatomic site (11) of region (1).

In order that the treatment be safe and penetrating, the stray E-fields produced by (5) are minimized according to the invention, and since the E-field between two points at distance $D$ (metre) of a circuit at voltage difference $V$ (Volt) is $E = V/D$ (Volt/metre) , such minimization implies first a low voltage difference across the terminals (12) and (13) which approximately define the active portion of (5). Being $V$ proportional to the inductive reactance $X_L \ (=2.\pi.f.L)$ of (5) at the operating frequency $f$, also $X_L$ must be low, which in turn implies either limited inductance $L$ values for (5) or low operating frequencies, or both. These conditions are fulfilled by the strip-inductor embodiments according to the invention with the operating frequency in the RF range and with the inductance $L$ of (5) kept low, and with imposed restrictions on the equivalent electric length $L_e$ of the tank circuit. In any case, any expert knows that both the electric length and the inductance of a circuit are lowered provided that the circuit is embodied with parallel winding sections or with small radius inductive structures (5), and this allows great freedrom in the choice of turn number and size within wide RF frequency sub-ranges. Moreover, a low E-field level between points (12) and (13) is obtained by positioning them far apart as much as possible and any residual E-field effect is minimized with both of them retracted from the body surface. This implies that the tank circuit loop including active strip 5 with retracted terminals (12) and (13), conductors (7) and network (4), lie across planes substantially perpendicular to the site surface (11).

Structures (5) according to the invention, hereinafter referred to either as strip-inductors or simply as inductors call be operated at frequencies up to 450 MHz and still preserving the required flexibility, selectivity and safety, and constructed as current-carrying conductive strips conformal to any anatomic site (11) either superficial or inside a body cavity. It appears evident to any expert that an inducing current (14) flowing across strip-inductor (5) exhibits unique features of tissue selectivity, precision, effectiveness and safety. First, it generates a short-range H-field (15) which is inducing heating RF current (14') producing core pattern (9') substantially following the strip-inductor (5) transverse layout. Moreover, the induced currents (14') are delivered with adequate intensity substantially only in the highly conductive muscle tissue layer (17), which is laying underneath the nonconductive adipous (16) and flow substantially parallel to the fat-muscle interface (16'), thus satisfying the safety condition. Thus, the heated core (9') is selectively localised in the subcutaneous or deep conductive tissues, i.e. in muscle, tendon, and interstitial fluids that are usually including targets, which, instead, are poorly heated with the prior art MW applicators.

FIG.s 2-12 show views with parts cut away of strip-inductor preferred embodiments in which shields and networks have been omitted. It is evident to any expert, that many further wire or sheet, single or multiple strip-inductors carrying currents of any intensity, frequency and phase, derived from those of FIG.s 2-12 by way of modifications, integrations and combinations may be embodied varying in size and shape and conformed to any site curvature, either superficial or within a body cavity, for the selective treatment of heterogeneous and variably perfused targets, of any shapes, sizes, depths and conformation, without departing from the scope of the invention.

FIG. 2 shows the S-shaped single wire strip-inductor preferred embodiment (18) delivering the core (9') that exhibits a narrow pattern following the strip-inductor wire layout, whilst FIG. 3 shows the C-shaped sheet strip-inductor embodiment (18a) delivering the core (9') exhibiting a semi-circular pattern cross-section overlaying the wide cross-section strip-inductor layout.

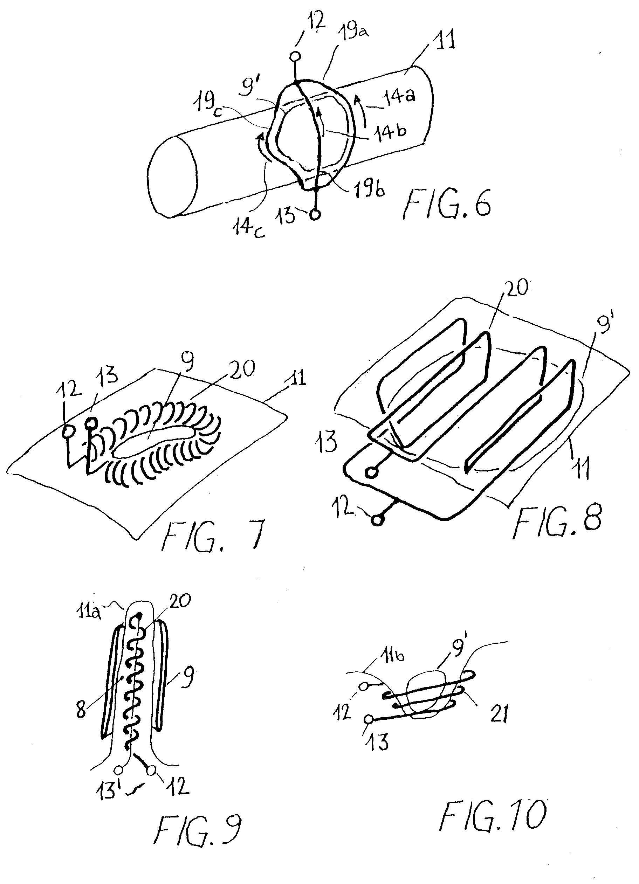

FIG.s 4,5 and 6 show preferred embodiments of multi-element inductors fed by coherent currents of controlled phase and amplitude, and embodied by a plurality of active strip inductors according to the invention. FIG.4 shows the preferred embodiment in which the phase-coherent RF currents (14a) and (14b) of substantially the same intensity and relative phase flow along the two angled straight strip-inductors (19a) and (19b), respectively, producing the substantially uniform trapezoidal core pattern (9') in the central region, owing to the constructive interference of the local currents induced by the two H-fields. FIG.5 shows instead the multi-element phased array embodiment in which the phase-coherent RF currents (14a) and (14b) of substantially same intensity but phase-opposed, flow along the two non symmetric concavity-opposed C-shaped strip-inductors (19a) and (19b), respectively, producing the two non-connected core patterns (9a') and (9b'), respectively, which are conformed to the curvature of the relative strip-inductor, owing to the destructive interference of the local currents induced by the respective H-fields flowing in the central region. It is apparent ot any expert that the same strip-inductors configured as in FIG.5 but fed with in-phase currents would, instead, give a central ellipsoidal core pattern due to constructive interference. It is also evident that the H-field phase reversal of any given strip-inductor obtained with a current reversal may also be obtained by the inversion of the strip terminals. FIG.6 shows the parallel split-element wire strip-inductors (19a), (19b) and (19c) embodiment which supports the parallel flow of the concurrent currents (14a), (14b) and (14c), and which is conformed to the curvature of site (11) to produce the conformal core (9') patterned according to the resulting strip layout configuration. More generally, multiple core patterns can further be obtained without departing from the scope of the invention with a plurality of strip-inductors of any size, shape and relative configuration, either series-connected or parallel connected, in which phase-coherent currents in definite intensity and phase relationships flow to give either connected or non-connected multiple interfering heating fields.

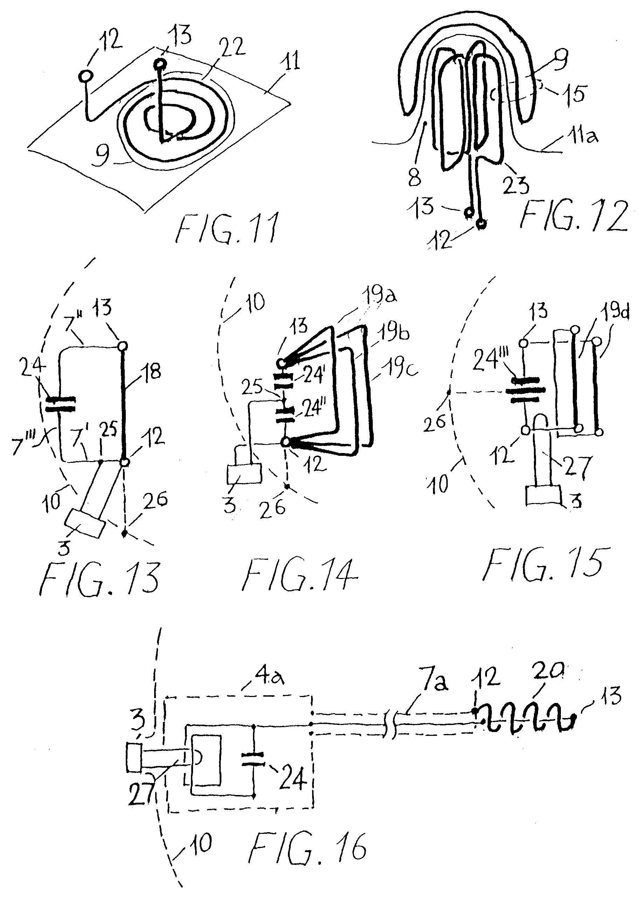

FIG.s 7,8,9 and 10 show some preferred embodiments for either superficial or endocavitary treatments of multi-turn strip-inductors according to the invention, hereinafter referred to as axial-inductors, consisting of small cross-sectional area coiled structures exhibiting a controlled inductance and equivalent electric length in which only the strips or the turn portion facing the tissue to be exposed are the active loopsides, so that the resulting core follows the configuration of the inductor axis. Axial-inductors generally exhibit a penetration proportional to the H-field intensity due to their inductance which depends on the turn transverse size, whereby the penetration of axial inductors within a small diameter cavity is smaller than that obtained with axial inductors designed for superficial treatments. It is evident to any expert that axial-inductors according to the invention can be embodied with any number of turns, wound with either uniform or variable pitch, with either tapered or stepped turn cross-section of any shape and size, and with either parallel or series connected winding sections, and may perform both paracorporeal and endocavitary treatments with any selected active parts of the turns. In FIG.7, the flexible axial-inductor embodiment (20) is patterned around the narrow superficial target (9) for a preferential heating of the target periphery, on account that the periphery is the highly blood perfused - and blood refrigerated - cancer advancing front requiring the delivery of higher EM energy levels than the non-perfused nucleus, that is heated by conduction to the therapeutic temperature. FIG.8 shows the split axial-inductor embodiment (20) consisting in a substantially rectangular two-section coil set of large cross section parallel connected, delivering the penetrating core (9'). FIG. 9 shows the uniform diameter and uniform pitch axial-inductor (20) preferred embodiment as long as the annular target (9) on the wall of cylindrical cavity (11a). In FIG.10, the tapered, annular end-fire axial-inductor (21) embodiment wraps protruding sites such as (11b), and could be used for either the endocavitary heating of the vault of the vaginal cavity for cervix tumor treatment, or for paracorporeal heating of protruding superficial targets.

Small-size strip-inductors in which the current carrying turns have their plane preferably laying conformally on the surface of site (11), are bi-dimensional strip-inductors a preferred embodiment (22) of which is schematically and reductively illustrated in FIG. 11 wound as a spiral, sized and shaped to overlay the small target (9). These bi-dimensional, small size inductor embodiments, hereinafter referred to as patch-inductors, are of low inductance and of low equivalent electric length, and may be shaped conformally to both superficial and endocavitary complex body sites.

A preferentially axial strip-inductor embodiment such as (23) of **FIG.12**, hereinafter referred to as a "toroidal-inductor", may be considered useful in endocavitary treatments. The current loops of (23) define a toroidal winding, whose magnetic fluxlines (25) leak from the toroid interturn spaces and impinge all around cavity (11a) wall.

It is evident to any expert that any strip-inductor according to the invention may be embodied of any cross section shape, size and pattern with any conductive wires or ribbons, or pipes in which refrigerating or heating fluid flow. Moreover, the air gap between any strip-inductor according to the invention and the body surface may be embodied with a variable thickness to produce a spatial modulation of energy delivery to the core along the strip-inductor layout according to target tissue etherogeneity, or depth, or tickness, or perfusion, without departing from the scope of the invention. Moreover, the insertion of a conductive shield which is partially hiding a tissue portion from a strip-inductor will help in limiting the core volume.

**FIG.s 13, 14, 15** and **16** show preferred embodiments of the disclosed tank circuit, in which details regarding strip inductors, shields and targets are omitted. **FIG.13** shows a tank circuit embodiment including single strip-inductor (18), the terminals (12) and (13) of which are connected to the tuning capacitor (24) via loopside conductors (7'), (7") and (7'"). The matching circuit of the network is embodied by tap (25) to conductor (7') at the cold terminal (12), which is adjusted with procedures well known to the experts to match the feeding cable at port (3) with minimal return loss. The cold terminal (12) may be grounded at (26) to shield (10). The inductive part of the tank circuit is the nearly rectangular loop including the front-side strip-inductor (18), the two loopsides (7') and (7") perpendicular to the strip-inductor (18) layout, and the back loopside (7'"), in which capacitor (24) is inserted, and that results retracted to help keeping away the capacitor stray E-field. This loop configuration guarantees that only strip inductor (18) is effective in heating the exposed tissue. It appears evident to any expert that tank circuits may be embodied with loops of any size and wit shapes different from the rectangular one, and not perfectly planar without departing from the scope of the invention. **FIG.14** shows the strip-inductor set preferred embodiment (19a), (19b) and (19c), parallel-connected at the common terminals (12) and (13), which is tuned by the two series-connected variable capacitors (24') and (24") which are operated as a standard matching circuit at the capacitive tap (25). **FIG.15** shows the series-connected two-loop strip-inductor (19d) embodiment. The stray E-fields due to the higher reactance of the series-connected loops is minimized with the help of the three-electrode tuning capacitor (24'") which is symmetrically grounded at point (26) of shield (10), and provides ground potential to strip-inductor (19d) central point, giving smaller stray E- fields from the medium-high inductance inductors and safely allowing the use of high power levels. The matching circuit is implemented by the loop (27) terminating the feeding line at the port (3) coupling to the inductive part of the tank circuit, which results galvanically isolated from the RF source. Smooth matching adjustements are performed by means of a rotatable or linear sliding joint, allowing loop (27) to assume different orientations or positions, respectively, with respect to the tank circuit. **FIG.16** shows, with parts cut avay, a preferred tank circuit embodiment for endocavitary applicators according to the invention, comprising axial-inductor (20) and network (4a), connected through the coaxial cable segment (7a) of length equal to the strip-inductor insertion depth in the cavity. Network (4a) includes tuning capacitor (24) and the matching loop (27) terminating the feeding cable in (3) which is coupled to the inductive part of the network. Being the RF frequency upper limit determined by the electric lenght $L_e$ of the tank circuit, whenever the electric length of one of its elements, be the inductor or the connecting mens or the network, increases, the other elements have to be embodied with shorter electric lengths. Since the endocavitary tank circuits exhibit generally long connecting cable segments, the operating frequency for endocavitary operation must be kept proportionally limited.

Tank circuits according to the invention can be embodied by any strip-inductor embodiments in conjunction with any connecting means embodiments and any textbook network embodiments, in many compatible combinations without departing from the scope of the invention. Furthermore, tuning means such as variable capacitors may be employed, although alternative tuning embodiments include scanning the source RF frequency.

The following optimization means, which are intended not to limit further combinations or extensions, may be embodied for the optimization of the heating fields to accomplish a precise and safe heating of any target under any condition without departing from the scope of the invention. Heating field controlling means according to the invention include strip-inductors embodied with mechanically flexible conductive wires or ribbons, bellows, or with plastic pipes filled with conducting fluids such as mercury, so that a variety of H-field sizes and shapes are obtained by adjusting the strip-inductor layout. Further H-field distribution optimization means according to the invention include adding to a first tank circuit directly energized by the RF power source, one or more passive tank circuits tuned to the same frequency, which are indirectly energized by the first tank circuit through preferentially magnetic couplings, to deliver a multiple core pattern.

To help controlling changes of the heating pattern during the treatment, heating field optimization means according to the invention include inserting in the air gap between the applicator and the body surface slabs of materials of specific conductive properties, in order to produce there induced RF currents to modify the tissue heating pattern distribution. Furthermore, slabs of magnetic materials may be inserted in the gap to modify the H-field in the tissue. Further control includes mechanical means modifying the configuration of flexible strip-inductors according to the required H-field pattern modifications.

The applicators according to the invention may be embodied with mechanical means providing cyclic motion along programmed pathways, to deliver adequate time-averaged power levels in the various parts of an estended target, a feature that cannot be implemented with the prior art MW applicators which require the coupling water bolus. Thus, conductive or magnetic slabs of the materials interposed in the gap may be mechanically scanned along programmed pathways. If required, frequency tracking means may keep the source frequency tuned to the tank circuit during the scans. A further scanning embodiment according to the invention include producing time-averaged heating field distributions with a multiplexer switching a single-output RF source to a multi-channel network circuit connected to a multiple strip-inductor set within the same applicator or in a multiplicity of applicators the individual strip-inductors of which are fed in sequence with RF pulses of controlled duty cycle.

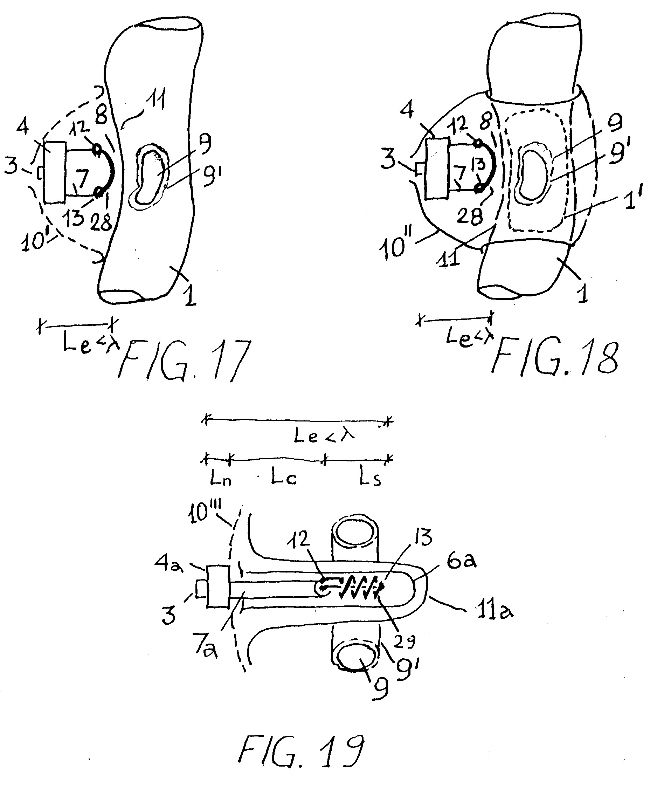

EM shields of variable efficiency according to the invention can be embodied with conductive means wrapping totally or partially the strip-inductors, or the whole tank circuit, for the total or partial containment of the EM energy within the shield boundary, respectively. **FIG.s 17,18** and **19** show some preferred shielding embodiments which increase the heating efficiency while reducing the stray EM radiation in the environment, and can furthermore be employed also as treatment adjuvants, and are, therefore, functional parts of the applicators according to the invention. In **FIG.17**, the target (9) is localised at the superficial site (11) presenting a curvature, and the paracorporeal strip-inductor (28) is conformed to site (11) and shaped and sized to overlay target (9) for a precise localized treatment. Shield (10'), hereinafter referred to as efficiency shield, is loosely wrapping the tank circuit and is impeding that RF radiation escapes through air gap (8), thus limiting stray EM fields whilst increasing the heating efficiency of strip-inductor (28). An efficiency shield (10') preferred embodiment is constituted by a conductive body loosely shaped over the tank circuit to which shielding wings are attached, which are embodied by preferably flexible single or multi-piece conductive flaps extending from the periphery of the shielding body to nearly contact the contour of the body surface. It is apparent to any EM expert that, without departing from the scope of the invention, to any strip-inductor embodiment may be associated an efficiency shield of non-critical size and shape and modeled according to the shape and size of the tank circuit to conform to body segments of any complex conformation. In **FIG.18**, the target (9) is embedded in the site (11) which is part of the body region (1), and the paracorporeal strip-inductor (28) is equipped with the integral shield (10"), hereinafter referred to as regional shield, that is wrapping loosely together the tank circuit and strip-inductor (28) with the region (1) in a quasi-perfect EM field containing enclosure, in which, however, the EM energy does not exhibit a standing wave character being the cavity dimensions smaller than the wavelength at the operating frequency. Thus, the body sub-region (1') encompassed by regional hield (10") is totally immersed in approximately the whole EM field produced by strip-inductor (28) and by the tank circuit, and heats up to sub-regional pedestal temperature $T_p$ lower than the therapeutic temperature $T_c$ of core (9'). Regional shield (10") helps to further enhancing the core (9') therapeutic temperature by further increasing the heating efficiency, while helping to sustain a more uniform distribution of temperature within the core with smaller temperature gradients at the target boundary owing to the established pedestal temperatures around the target, a result of clinical relevance in cancer therapy. Moreover, regiona shields (10") help producing a beneficial regional hyperhemia across the sub-region (1'), which is of clinical relevance in rehabilitation treatments. It is apparent to any expert, that all the efficiency and regional shields according to the invention can be embodied with any conductive material, preferably flexible and of low density, including thin metallic sheets, fabrics or non-conductive sheets sprayed with conductive paints, metallic nets with openings substantially smaller than the operating wavelength. Moreover, regional shields (10") according to the invention can be embodied to wrap tank circuits equipped with any type of paracorporeal strip-inductor and any body region of any complex conformation, on account that that their size and shape are not critical.

In **FIG.19**, a preferred tank circuit embodiment according to the invention is schematically illustrated for the endocavitary treatment of target (9) localised across the the wall of body cavity (11a). An endocavitary strip-inductor reductively illustrated as axial inductor (29), is supported by the remote head of the flexible catheter (6a), and is sized and shaped to be conformed to cavity wall, producing the annular core (9'). The network (4a) is positioned externally at the cavity opening and the coaxial cable segment (7a) external shield and central conductor are connected to the cold and hot terminals (12) and (13) of strip-inductor (29), respectively. At frequencies close to the upper limit of the RF range, the cold terminal (12) may alternatively be connected to the cable (7a) outer shield by a capacitive coupling. The outer shield (10'") is loosely wrapping the body site around the cavity aperture, hereinafter referred to as EM shield, whilst it has a non-significant effect on heating efficiency, and provides a boundary of non-critical size for the residual EM field of (29) leaking from the body in the environment, being furthermore functional as a ground plane to a stable operation of strip-inductor (29).

It will be apparent to any electromagnetic hyperthermia expert, that the heating method and the applicators embodiments illustrated in **FIG.s 2-19**, and those derived from them by estensions, modifications and combinations, fall within the scope of the invention, and are suited to perform localized HT treatments of any target on any anatomic site, either superficial or subcutaneous or else inside a body cavity, and are more safe and practical than those of the prior art , owing to their not requiring to be at direct-contact with the patient and not producing stray E-fields, whilst exhibiting improved precision in tayloring heating fields which can be optimized on both site and target.

**Claims**

1. An electromagnetic heating method for the localized hyperthermic treatment to the therapeutic temperature of a tissue target of any shape, size and conformation, and localized in any anatomic site of any region of the body by exposing said target to the RF energy beam produced by an applicator fed by a power source generating said RF energy at an operating frequency in the 6-450 MHz range, said method comprising;

   - providing a tank circuit comprising both strip-inductor means and capacitive tuning means together with conductive means interconnecting said strip inductor means with said capacitive means;
   - providing matching means to match said tank circuit to said power source;
   - providing positioning means for said strip-inductor means and partial electromagnetic field director means to directly convey said RF energy beam towards said target through an air gap to produce an heating core encompassing said tissue target;
   - providing optimization means for said RF energy beam for helping to produce said heating core of size, conformation and depth precisely encompassing said target said optimization means comprising a multi-element strip-inductor set, each of said elements being adjusted in its individual size, shape and air gap and carrying an RF current of controlled phase and amplitude, said elements being further adjusted in their relative configuration in order to modify the contribution of their mutual interactions to said heating core;
   - providing means to minimize the generation of electric fields from said strip-inductor set by controlling the inductive reactance of said strip-inductor set;
   - providing means to minimize the electric field intensity on the surface of said body generated by said capacitive tuning means by withdrawing said capacitive means from the said surface of said body;
   - providing means to minimize the intensity of the electric fields on the surface of said body generated by said interconnecting means by withdrawing said interconnecting means from said body surface and avoiding to lie them parallel to said body surface;
   - providing EM enclosure means contributing to the stability of said tank circuit and to improve the heating efficiency by limiting the stray EM field levels;
   - adjusting said tuning capacitance until said tank circuit is tuned to the operating frequency and adjusting said matching means until the input port of said tank circuit is matched to the output impedance of said power source;
   - providing control means for the RF energy level of said power source to control said therapeutic temperature of said core.

2. The heating method as claimed in claim **1** wherein said EM enclosure means include providing a quasi-perfect EM enclosure circumscribing said tank circuit to peripherically occlude also said air gap in order to preclude the leakage of part of said RF energy through said air gap.

3. The heating method as claimed in claim **1** wherein said EM enclosure means include providing a perfect EM enclosure circumscribing said tank circuit together with said body region to peripherically occlude also said air gap in order to preclude a substantial leakage of part of said RF energy through both said body region tissue and said air gap.

4. An electromagnetic applicator for the hyperthermic treatment to the therapeutic temperature of a tissue target localized across any anatomic site of any region of the body by exposing said target to the RF energy beam produced by said applicator, said applicator being fed by an RF power source, the frequency of said source ranging from about 6 MHz to 450 MHz, said source delivering said RF energy to said applicator through a feeding cable, said applicator comprising;

   - a radiating system constitutted by a set of strip-inductors producing said RF magnetic field beam made available as therapeutic field coupled magnetically through an air gap to said body region exposed to said strip-inductors through air gaps;
   - capacitive means for tuning said strip-inductor set to produce a tank circuit operated in said frequency range, said capacitive means being electrically connected to said strip-inductors of said set by a plurality of connecting conductors, said conductors being configured to lay along directions substantially perpendicular to the layout of said strip-inductors;
   - matching means to match the impedance of said tank circuit to that of said power source;

- means for adjusting said magnetic field beam to specific size, shape, orientation and depth in order to help producing an heating core encompassing said tissue target, said adjusting means including changing the cross-section shape, size and conformation of the layout of said strip-inductors set together with the respective tickness of said air gaps;

- means for minimizing the residual electric field of said strip-inductors on the surface of said body compatibly with the generation of said heating core, said minimizing means including adjusting the inductive reactance of said set of strip-inductors through adjustements of the electric connections, the magnetic couplings, and the said air gaps of both said strip-inductors and said interconnecting conductors;

- EM enclosure means embodied with conductive means acting as EM shielding means to help reducing the stray EM radiation;

- means for adjusting the size, shape and conformation of said EM enclosure means for controlling the shielding effectiveness.

5. The applicator as claimed in Claim **4** wherein said means for adjusting the size, shape and conformation of said EM enclosure comprises constructing a quasi-perfect EM enclosure with said conductive means electrically insulated from said tank circuit and from the surface of said body region, said enclosure circumscribing said tank circuit with its electrically insulated periphery contacting the surface of said anatomic site all around said tank circuit to occlude also said air gap in order to preclude the leakage of part of said RF energy through said air gap.

6. The applicator as claimed in Claim **4** wherein said means for adjusting the size, shape and conformation of said EM enclosure comprises constructing a perfect EM enclosure with said conductive means electrically insulated from both said tank circuit and the surface of said body region, said enclosure circumscribing said tank circuit together with said body region, the electrically insulated periphery of said enclosure contacting all around the surface of said body region to occlude also said air gap in order to preclude a substantial leakage of part of said RF energy through both said body region tissue and said air gap.

7. The applicator as claimed in Claim **4** wherein said means for adjusting said beam to a specific orientation comprise partially limiting the angular spread of said beam by placing inner conductive electromagnetic shields partially surrounding said strip-inductor set.

8. The applicator as claimed in Claim **4** wherein said means for adjusting said beam to specific cross-section size and shape and orientation and depth, include sizing and shaping the cross-section of said strip-inductor set layout to overlay the size and shape of said target, and conforming the layout of said strip-inductor set to the conformation of said anatomic site.

9. The applicator as claimed in Claim **4** wherein said means for adjusting said beam, to specific size shape, orientation and depth include constructing said strip-inductor set with a plurality of discrete strip-inductors forming a discrete multi-element radiator and allowing coherent RF currents flowing in each of said discrete element, said RF currents being adjusted in phase and amplitude for controlling the extent of the interfering processes and the localization and depth of said heating core together with the temperature enhancement of said core.

10. The applicator as claimed in Claim **4** wherein said means for adjusting said beam to specific size, shape, orientation and depth include constructing said strip-inductor set with distributed-current strip-inductors, each said distributed-current strip-inductor being constructed with a conducting sheet in which a continuum of phase and amplitude adjusted coherent RF current lines flow to help producing said heating core controlled in localization, penetration, and temperature enhancement.

11. The applicator as claimed in Claim **4** wherein said capacitive tuning means are constructed out of printed circuit boards in which the dielectric layer interposed between the two conductive cladding sheets exhibits low RF dielectric losses.

FIG.1

FIG. 2

FIG. 3

FIG. 4

FIG. 5

FIG.6

FIG. 7

FIG.8

FIG.9

FIG.10

FIG. 11

FIG. 12

FIG. 13

FIG. 14

FIG. 15

FIG. 16

FIG. 17

FIG. 18

FIG. 19